# EUROPEAN PATENT APPLICATION

(11) **EP 4 400 123 A1**
(43) Date of publication of application: **17.07.2024**
(21) Application number: 23382008.3
(22) Date of filing: 10.01.2023
(51) Int. Cl.: A61L 9/03, A61L 9/04, B05B 17/06

(54) **STOPPER AND WICK ASSEMBLY FOR DEVICES FOR DIFFUSING VOLATILE SUBSTANCES**

(71) Applicant: Zobele Holding SpA, 38123 Trento (IT)
(72) Inventor: DEFLORIAN, Stefano, 38123 Trento (IT); MORHAIN, Cedric, 08019 Barcelona (ES)
(74) Representative: Herrero & Asociados, S.L.

(57) **Abstract**

The stopper and wick assembly for devices for diffusing volatile substances comprises a wick (2) for diffusing the volatile substances; and a stopper (1) placed around the wick (2), covering a portion of the wick, wherein the wick (2) and the stopper (1) are bonded to each other.

It permits to provide a stopper and wick assembly for devices for diffusing volatile substances in which the stopper is bonded to the wick in a non-removable way.

## Description

The present invention relates to a stopper and wick assembly for devices for diffusing volatile substances, wherein the stopper and the wick are bonded in a non-removable way.

### Background of the invention

Volatile substances diffusers of many kinds are available on the market for years. One feature of these kinds of devices is that they have a wick or capillary means that transports the liquid from a container to the environment.

Emanation could happen by evaporation or diffusion, passively, or through the effect of a heater and/or a fan, or by nebulization for example through an ultrasonic actuator.

There are several technologies also known for years about the kind of capillary means or wicks to be used: ceramic, porous plastic, plastics fibers, porous wood, etc. Sometimes these wicks come with some external wrap for controlling better the evaporation or even simply to give the body of the wick. An example of this last kind of wick is like cigarette filters, where a bundle of fiber is wrapped in a paper wrap.

Another element often present when you a wick is present is a stopper. The stopper is a plastic element, generally with a cylindrical geometry similar to the container neck, that bonds the wick to the container neck. Stopper used to be inserted in the bottle neck with a high stress level, that makes impossible to extract it from the bottle without tools, while the wick being quite a soft material, it cannot generate a high level of stress with the stopper and is quite easy to remove.

Often it is a must that the wick cannot be easily removed from the container or if the wick is removed, it cannot be replaced, i.e., it cannot be reinserted. The target of this is to avoid refilling the container with whatever liquid. The original liquid is a safe liquid that has been evaluated and confirmed in order to guarantee health and security of the user.

Some users may have the skill to understand what other liquid can be used and what should not, but this will not be the majority of users.

Some solutions are known that guarantee this bonding of the wick and the stopper. For example, a metallic nail that passes through the stopper and the wick; or a special design of the stopper with fingers that allow the wick to be introduced in the stopper in one direction but does not allow the wick to be moved in the other direction.

In some other solutions, the wick comprises a 3D shape, for example, a ring that cannot pass through the stopper. While typical cylindrical wicks are made with a continuous process, such as extrusion or wrapping of a bundle, this kind of 3D shape is done one by one, and its cost makes the product less competitive.

Therefore, current solutions present several drawbacks:
For example, a metallic nail through the stopper and the wick is a strange body that will complicate possible recyclability of the plastics. Then, the nail needs to be inserted before the wick and stopper are placed inside the container.

The containers, often made of glass, have big tolerances in dimensions and so the depth of the container can change a lot in a same batch, so that often there is a gap between the bottom of the wick and the bottom of the container.

One way stopper still has the trick that the stopper can be removed, extracting the wick by pulling it in the same direction it was introduced and then reuse it.

3D shape wicks have the same issue, if you can remove the stopper then you can remove the wick and reuse it, apart the higher cost already explained.

### Description of the invention

Therefore, an objective of the present invention is to provide a stopper and wick assembly for devices for diffusing volatile substances in which the stopper is bonded to the wick in a non-removable way.

With the stopper and wick assembly for devices for diffusing volatile substances of the invention, said drawbacks are solved, presenting other advantages that will be described below.

The stopper and wick assembly for devices for diffusing volatile substances according to the present invention is defined in claim 1. The dependent claims include additional features, which are optional.

The stopper and wick assembly for devices for diffusing volatile substances according to the present invention comprises:
- a wick for diffusing the volatile substances; and
- a stopper placed around the wick, covering a portion of the wick,
wherein the wick and the stopper are bonded to each other.

Preferably, the wick and the stopper are welded to each other.

According to a preferred embodiment, the wick comprises a central portion made from fibers and a skin surrounding the central portion of the wick, the material of the skin being different from the material of the fibers.

Furthermore, the material of the central portion of the wick has a higher thermal resistance than the skin of the wick.

Advantageously, the wick can comprise one or more bonding segments, by which the wick is bonded to the stopper.

For example, the one or more boding segments extend over a length that is at least a 25 % of the perimeter of the wick, and the depth of the one or more bonding segments inside the wick is up to a 15 % of the thickness of the wick.

According to a preferred embodiment, the stopper comprises a ring and a neck, and optionally a skirt, and preferably, the wick is bonded to the stopper by the neck or the skirt, if present.

### Brief description of the drawings

For a better understanding of what has been stated, some drawings are attached in which, schematically and only as a non-limiting example, a practical case of embodiment is shown.

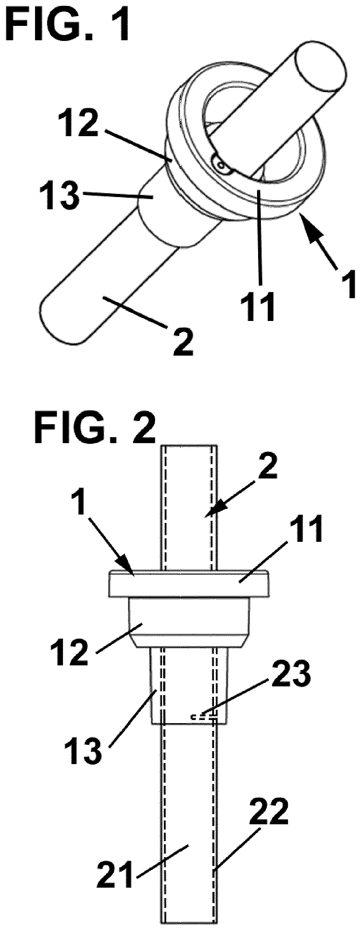

Figure 1 is a perspective view of the stopper and wick assembly for devices for diffusing volatile substances according to the present invention; and
Figure 2 is an elevation view of the stopper and wick assembly for devices for diffusing volatile substances according to the present invention.

### Description of a preferred embodiment

The stopper and wick assembly for devices for diffusing volatile substances according to the present invention comprises a stopper 1 and a wick 2, said wick 2 crossing the whole stopper 1, as shown in the drawings.

The stopper 1 comprises a ring 11, which in the use position is placed protruding from a neck of a container (not shown in the drawings), a neck 12, which in the use position is placed inside the neck of the container and is leakage proof, and also avoids the removal of the stopper from the container due to high friction level, and a skirt 13, which is optional, placed around the wick 2.

The wick 2 is bonded to the stopper 1 in a non-removable way, such as, e.g., by welding.

The welding between the stopper 1 and the wick 2 has some requirements, because the wick 2 is usually made by soft materials and the welding process requires the wick 2 to be able to withstand a certain pressure during the welding, i.e., to provide a counter pressure during welding. Furthermore, the main function of the wick 2, capillarity, shall not be affected by the welding process.

Therefore, the wick 2 and the stopper 1 are bonded, e.g., by welding or fusion bonding, in at least one point of the interface between the wick 2 and the stopper 1. In this context, fusion bonding means that, using two materials with one of them that is porous, the other material melts and flows in the pores and then get solid in the pores, so that two materials are joined that would not weld if they were in a non-porous film state.

Preferably, the area or point affected in the wick 2 by this welding has a limited depth, preferably below 1 mm. Still preferably, the welding or fusion bonding is done at the level of the bottom part of the stopper 1.

So, to do such bonding, the material of the wick 2 and the stopper 1 need to be compatible to create a non-removable bonding with the stopper 1 by welding, so that preferably the fiber material of the wick 2 is of the same kind than the material of the stopper 1.

Alternatively, the external surface of the wick 2 has a porosity that allow the material of the stopper 1 to penetrate inside the pores of the wick 2 during the welding process and then be mechanically clamped, i.e., fusion bonded, in the pores of the wick 2.

Preferably, the wick 2 comprises a central portion 21 with fibers and an outer surface or skin 22 with a material that is different from the fibers and compatible with the stopper 1 for welding or fusion bonding.

Advantageously, the material of the central portion 21 of the wick 2 has a higher thermal resistance (melting or softening point) than the skin 22 of the wick 2. This way, the alteration of the capillary properties will be limited to the skin 22 and the properties of the central portion 21 are not affected.

Any suitable material could be used for the stopper 1 and the wick 2, but preferable material is a polyolefinic based material, such a PP or LDPE, HDPE, UHDPE or copolymers.

Just as one example, the stopper 1 can be made from Low Density Polyethylene (LDPE), and the central portion 21 of the wick 2 can be made from polyethylene terephthalate (PET) fibers that are gathered and maintained together with the skin 22, which is preferably porous, allowing the liquid/vapor to pass through to allow evaporation.

This skin 22 can be made from a polyethylene based non-woven, that is bonded to the stopper 1 by welding. The polyethylene based non-woven can be made from bicomponent fibers of PET/polyethylene, where PET is the core of the fiber and polyethylene is the skin 22.

Alternatively, the wrap can be made from paper that will be bonded to the stopper 1 by fusion bonding.

According to preferred embodiments, which are not limitative, the stopper and wick assembly according to the present invention can be made by the following methods.

The stopper 1 and wick 2 are preferably assembled and bonded together before their introduction in the container.

Firstly, the wick 2 is introduced inside the stopper 1, and two heating plates can press each side of the stopper 1.

Even though it is a quite aggressive process that apply a lot of heat on the stopper 1 from outside to inside, it is an option for bonding the wick 2 to the stopper 1.

A stopper 1 having a different composition on the inner side, like a lacquer or a coextruded layer, that has a lower melting point than the material of the outside can also be used, in such a way the inner material can be melt without melting the outer one that is the one which is pressed.

An alternative option can be to preheat the interface between the wick 2 and the stopper 1 by using a laser beam, for example, and, in a second step, to apply pressure on both sides of the stopper 1 with non-heated plates.

For obtaining the required bonding between the wick 2 and the stopper 1, a suitable amount of the perimeter of the interface between the wick 2 and the stopper 1 must be welded, with one or more bonding segments 23.

For example, said one or more bonding segments 23 can extend over a total length of 25 % or more of the perimeter of the wick 2.

The depth of the bonding segment(s) 23 inside the wick, that might substantially reduce or compromise the capillarity is limited to be not more than 15 % of the thickness of the wick 2.

Even though reference has been made to a specific embodiment of the invention, it is clear to a person skilled in the art that the described device for diffusing volatile substances is susceptible to numerous variations and modifications, and that all the mentioned details can be substituted by others technically equivalent ones, without departing from the scope of protection defined by the appended claims.

## Claims

1. Stopper and wick assembly for devices for diffusing volatile substances, comprising:
- a wick (2) for diffusing the volatile substances; and
- a stopper (1) placed around the wick (2), covering a portion of the wick,
**characterized in that** the wick (2) and the stopper (1) are bonded to each other.

2. Stopper and wick assembly according to claim 1, wherein the wick (2) and the stopper (1) are welded to each other.

3. Stopper and wick assembly according to claim 1 or 2, wherein the wick (2) comprises a central portion (21) made from fibers and a skin (22) surrounding the central portion (21) of the wick (2), the material of the skin (22) being different from the material of the fibers.

4. Stopper and wick assembly according to claim 3, wherein the material of the central portion (21) of the wick (2) has a higher thermal resistance than the skin (22) of the wick (2).

5. Stopper and wick assembly according to any one of the previous claims, wherein the wick (2) comprises one or more bonding segments (23), by which the wick (2) is bonded to the stopper (1).

6. Stopper and wick assembly according to claim 5, wherein the one or more bonding segments (23) extend over a length that is at least a 25 % of the perimeter of the wick (2).

7. Stopper and wick assembly according to claim 5 or 6, wherein the depth of the one or more bonding segments (23) inside the wick (2) is up to a 15 % of the thickness of the wick (2).

8. Stopper and wick assembly according to any one of the previous claims, wherein the stopper (1) comprises a ring (11) and a neck (12).

9. Stopper and wick assembly according to claim 8, wherein the stopper (1) also comprises a skirt (13).

10. Stopper and wick assembly according to claim 8 or 9, wherein the wick (2) is bonded to the stopper (1) by the neck (12) or the skirt (13).
